(19)

**Europäisches Patentamt**

**European Patent Office**

**Office européen des brevets**

(11)  **EP 2 207 806 B1**

(12)  **EUROPEAN PATENT SPECIFICATION**

(45) Date of publication and mention
of the grant of the patent:
**10.04.2013 Bulletin 2013/15**

(21) Application number: **08844541.6**

(22) Date of filing: **29.10.2008**

(51) Int Cl.:
***C07K 16/18*** *(2006.01)*   ***A61K 47/48*** *(2006.01)*

(86) International application number:
**PCT/EP2008/009110**

(87) International publication number:
**WO 2009/056280 (07.05.2009 Gazette 2009/19)**

(54) **ANTIBODIES WHICH BIND SELECTIVELY TO HAIR OF ANIMALS AND AN ANTIBODY BASED DRUG DELIVERY SYSTEM FOR ANIMALS**

ANTIKÖRPER MIT SELEKTIVER BINDUNG AN TIERHAARE UND WIRKSTOFFAUSGABESYSTEM AUF ANTIKÖRPERBASIS FÜR TIERE

ANTICORPS SE LIANT SÉLECTIVEMENT AUX POILS D'ANIMAUX ET SYSTÈME D'ADMINISTRATION DE MÉDICAMENT À BASE D'ANTICORPS POUR ANIMAUX

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **02.11.2007 EP 07021396**

(43) Date of publication of application:
**21.07.2010 Bulletin 2010/29**

(73) Proprietor: **Bayer Intellectual Property GmbH 40789 Monheim (DE)**

(72) Inventors:
*   **HOFMANN, Stefan**
    **40764 Langenfeld (DE)**
*   **HAMANN, Hans-Juergen**
    **41539 Dormagen (DE)**
*   **FISCHER, Rainer**
    **52156 Monschau (DE)**
*   **SCHILLBERG, Stefan**
    **52074 Aachen (DE)**
*   **VOGEL, Stefan**
    **52066 Aachen (DE)**
*   **SCHINKEL, Helga**
    **52074 Aachen (DE)**

(74) Representative: **BIP Patents c/o Bayer Intellectual Property GmbH Creative Campus Monheim Alfred-Nobel-Straße 10 40789 Monheim (DE)**

(56) References cited:
**US-A- 3 987 161    US-A- 5 425 937 US-A- 6 123 934**

*   **PALUZZI S ET AL: "Anti-keratin Monoclonal Antibodies for Identifying Animal Hair Fibers" TEXTILE RESEARCH JOURNAL, SAGE PUBLICATIONS, LONDON, GB, vol. 74, no. 5, 1 January 2004 (2004-01-01), pages 458-464, XP002448425 ISSN: 0040-5175 cited in the application**
*   **MCCARRON PAUL A ET AL: "Antibody conjugates and therapeutic strategies" MOLECULAR INTERVENTIONS, AMERICAN SOCIETY FOR PHARMACOLOGY AND EXPERIMENTAL THERAPEUTICS, BETHESDA, MD, US, vol. 5, no. 6, 1 December 2005 (2005-12-01), pages 368-380, XP002498492 ISSN: 1534-0384**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention provides antibodies which bind selectively to hair of animals and an antibody based drug delivery system in which a particular formulation can be directed to animal hair. This is driven by the need of increasing the effective period of therapeutics for animal ectoparasites treatment, reducing the toxicity of these drugs and improving their release profile.

Two monoclonal antibodies were selected showing high binding to the surface of dog hair. Importantly, no binding to human, horse, or cat hair was detectable. The data presented here demonstrate that the selected antibodies are suitable for designing a delivery system that directs drugs to animal hair.

**Prior Art**

Attachment or linkage of antibodies to hair structures and beneficial use of those antibodies to link hair treatment actives or particles containing hair treatment actives has been known to the world of those skilled in the art since quite a long time. Widder (US Patent 3,987,161) describes a hair care product comprising an antibody containing serum which has been obtained from an animal which is capable of forming antibodies in the blood when its body is injected with an aqueous suspension of mammalian, preferably human hair. Igarashi et al. (US Patent 5,597,386) describes an anti-hair antibody carrying hair dye or polymer particles; e.g. polystyrene particles, containing coloring substances, thus binding the coloring actives to human hair in order to obtain benefits in hair treatment. The antibody is described as an anti-keratin antibody which has been obtained by injection of keratin polymer intramuscularly to mammals and gaining the antibody via colostrum milk, where the keratin was obtained after hydrolysis and dissolution of human hair structures. The dye-carrying particles are described as macromolecular carriers. Koyama et al. (US Patent 6,123,934) describes cosmetic compositions containing an antibody binding to hair and linking latex particles of a polymer or a copolymer of a vinyl monomer to the hair structure by use of this antibody. The formulations may contain cationic polymers or other ingredients to improve or repair hair structure. In this invention, the antibody has been obtained by immunizing poultry by injection of hydrolyzed human hair or particles of human hair which had been obtained by powdering whole hair fiber structures. In all of those inventions, human hair structures are used for immunization and keratin binding polyclonal antibodies have been obtained. In general, it is known that antibodies can be generated by immunization of mammals or birds with biological antigen structures which may then be used to direct beneficial actives which are linked directly or indirectly to those antibodies to the areas of need, e.g. cancer cells.

Paluzzi and co-workers (2004) have used type II keratins from cashmere as antigens to produce species-specific monoclonal antibodies. Selected antibodies were tested by two-dimensional immunoblotting for immunoreactivity with keratins isolated from cashmere and wool. Several quantitative and qualitative differences were detected enabling the specific identification of cashmere when compared to other animal fibers. However, selected monoclonal antibodies bind to all samples and only minor differences in signal intensity and protein pattern were observed between the tested species. Importantly, antibodies bind to extracted keratin type I and II proteins - binding to the hair surface has not been demonstrated.

In the veterinary field, one may benefit from those inventions by using particles containing a beneficial agent and target those agents to hair keratin structures or alternatively skin structures by using an animal hair or animal skin binding antibody. This is especially useful if one considers the option to use these particles as bodies which may release those drugs for a prolonged period of time, i.e. for weeks, month or even one year, therefore protecting the animal against pest animals, namely insects, e.g. fleas and ticks, or making use of other beneficial effects, e.g. repelling pest insects, or curing skin diseases for this long period of time. Typical formulations designed for this purpose are prepared as a one time application, showing the beneficial effect from several hours to approximately 4 weeks. Using carriers systems like polymer particles may provide the option to extend this period of time considerably. Using antibodies to link those particles to hair may provide an option to provide a linkage of the active to the hair which is sustainable enough to allow the drug carriers to stick to the hair or skin for the time period which is needed. Suitable formulations are needed. Application systems which may be useful to apply those formulations to the animal are, e.g. sprays or so called spot-on or wipe-on formulations, which bring the formulation in touch with the animal skin or fur. Actives of interest may be e.g. pyrethroids or insecticides in general, as well as systemic drugs or behavior modifying agents.

The methods described in the patents above may provide access to such drug delivery systems. However, as the antibodies described in those inventions and gained by the described methods bind to hair keratin structures, one major disadvantage appears: By applying the formulations to the animal via a spray or spot-on formulation, a strong and sustained binding to mammal hair, namely keratin structures will be obtained regardless of the species present. Therefore, the human applicator may also receive doses of actives by accidentally binding those drug carrying systems to body hair or head hair. Also, by stroking the animal, a transmission of those drug carrying actives to the human body may be possible, which might expose the pet owners to sometimes aggressive actives, e.g. pyrothroids. For application safety reasons, it would be a big advantage if one could obtain antibodies which allow to target the hair or skin of certain animal species selectively. It is also of considerable advantage to have such kind of antibody formulations available if the veterinarian has to use those formulations in stables where multiple species are kept and only one species shows the disease to be cured or only one species has to be protected from a species-specific pest.

If one uses the state-of the art methods described above to obtain antibodies to animal hair the person skilled in the art would expect to gain antibodies binding to keratin structures. Keratin is common to all mammals and protein sequences between different species are much conserved. Therefore, anti-keratin antibodies will bind to mammal hair in general.

In the present invention, surprisingly, it was found that species-specific hair binding antibodies could be obtained which bind only to species-specific animal hair surface structures, not to human hair surface or hair surface of a different species than the one whose hair had been used for immunization. Obviously, species specific antigen structures exist which can be targeted by those antibodies. A drug carrying formulation comprising those antibodies and drugs which are linked to those antibodies as molecular entity or packed into drug containing particles is able to avoid the disadvantages described above.

Description of the embodiments of the present invention

The present invention provides an antibody which selectively binds to the surface of hair of one animal species selected from the group of dog, cat, cattle, sheep, goat, camel, llama and horse.

Such an antibody is selected from the group comprising polyclonal antibodies, monoclonal antibodies or recombinant antibodies such as full-size antibody, dimeric secretory IgA, multimeric IgM and fragments thereof such as F(ab')$_2$-fragment, Fab-fragment, Fv-fragment, single chain Fv antibody (scFv), bispecific scfv, diabody, triabody, tetrabody, dingle domain antibody (dAb), minobody or molecular recognition unit (MRU), derived from hybridoma cells, synthetic, semi-synthetic, naïve and immunocompetent phage libraries or ribosome display libraries, or by the generation of fully synthetic designer antibodies.

The present invention further provides a drug delivery system to target an individual animal species, comprising the antibody, having the ability of binding selectively to the hair surfaces of said individual animal species.

Said delivery system comprising the antibody bound or attached to the surface of solid particles, thus selectively binding these particles to animal hair surface structures.

The drug delivery system of the present invention comprises of a formulation of the antibodies selectively binding to animal hair of individual species and particles containing an active ingredient, in such a way that the antibody is binding selectively to hair of said individual animal species in a first step and the particles bind to the antibody coupled to the animal hair in a second step.

Further the drug delivery system of the present invention comprises a formulation of the animal hair binding antibody linked or chemically bound to a suitable spacer/mediator/intermediate coupling agent and of particles, in such a way that the spacer/mediator/coupling agent carrying the antibody becomes selectively bound to animal hair via said antibody in a first step and that the particles become linked to the animal hair by binding to said spacer/mediator/coupling agent in a second step.

The present invention further provides a drug delivery system comprising a formulation of the animal hair binding antibody and of particles to which surface a suitable spacer/mediator/intermediate coupling agent is linked or attached, in such a way that the antibody selectively binds to animal hair in a first step and the particles carrying the spacer/mediator/coupling agent are linked to animal hair by binding to said antibodies in a second step.

The drug delivery system according to the present invention comprises the antibody selectively binding to animal hair and particles in a way that the antibody and the particles may be present in a single formulation which is applied to the animal or may be present in two different formulations (I) and (II) which becomes mixed shortly before application to the animal or, alternatively, may be present in two different formulation (I) and (II) in such a way that formulation (I) is applied to the animal in a first step and formulation (II) is applied to the animal shortly after in a second step.

The drug delivery system comprises particles which may or may not contain active ingredients or agents beneficial for the animal, namely drugs, insecticides, hair or skin caring agents, repellent agents which protect the treated animal from being attacked by harmful pest animals, e.g. insects, smell modifying agents or agents modifying the behavior of the treated animal or other non treated individual animals which interact with the animal to which the treatment is applied.

The particles may contain active ingredients or agents beneficial for the animal in a concentration suitable for its therapeutic or beneficial purpose, namely 0.01% to 99.9%.

The drug delivery system according to present invention is a system in which the particles itself contain the active ingredients or agents beneficial for the animal, namely drugs, insecticides, hair or skin caring agents, repellent agents which protect the treated animal from being attacked by harmful pest animals, smell modifying agents or agents modifying the behavior of the treated animal or other non treated individual animals which interact with the animal to which the treatment is applied.

The particles may have a diameter of 0.001$\mu$m to 10$\mu$m, preferably 0.1$\mu$m to 2$\mu$m.

The drug delivery system of the present invention, after a single treatment has been applied to the animal, allows the protection of the said animal against diseases, a continuing therapy of animal diseases or which allows the animal to benefit from the effects of said actives for a prolonged period of time, namely ranging from several hours to 365 days.

The drug delivery system of the present invention further comprises a formulation of an antibody selectively binding to animal hair and an agent beneficial to an animal in such a way that the beneficial agent is linked/coupled/ bound to said antibody either directly or via a spacer/mediator/coupling agent in such a way that the linkage of said active to said antibody or spacer/mediator/coupling agent becomes broken by the environmental conditions of the animal fur or skin, thus releasing the active or part of the active or part of the active-antibody structure to perform its effect beneficial to the animal.

The drug delivery system acts in such a way that the breakage of the bonding of the active to the antibody or spacer/mediator/coupling agent happens randomly for a prolonged period of time, allowing the protection of the said animal against diseases, a continuing therapy of animal diseases or allowing the animal to benefit form the effects of said actives for a prolonged period of time, namely ranging from several hours to 365 days.

The drug delivery system as described here comprises an antibody specifically binding to animal hair or a formulation of said antibody, wherein said antibody binds to animal hair with a suitable binding strength in such a way that the amount of antibody and the amount of active ingredient attached to said antibody binding to animal hair remains large enough to maintain the desired beneficial effects for a prolonged period of time, even after the animal fur has been in contact with water for several times, e.g. by bathing or exposure to rain, or after the animal has been washed with surfactant containing solutions.

An antibody according to the present invention is able to maintain its selective binding capacity and specificity to animal hair even if it is kept at 4°C in a solution containing water and up to 50% of organic water miscible solvents up to a period of 24h.

The antibody is further able to maintain its selective binding capacity and specificity to animal hair even if it is formulated or kept in aqueous solutions containing other hair treatment compounds, namely surfactants, nonionic, anionic or cationic polymers and water miscible alcohols in concentrations which are typically applied in hair care formulations.

In a preferred embodiment the drug delivery system comprises the inventive antibody which is in addition bound or attached to the surface of solid particles, either directly or via a suitable spacer/mediator/intermediate coupling agent, thus selectively binding these particles to animal hair.

Description of the Figures

Figure 1 shows the binding of antibodies B7.3 and B18-4.3 on human and dog hair. Binding of the monoclonal antibodies to hair of various dog breeds and to human hair using the microtiter plate filtration ELISA with 5 ng of antibody on 1 mg hair/well. Detection with goat-anti-mouse Fc coupled to horseradish peroxidase and ABTS-substrate. All samples were analysed in three replicates and standard deviations were calculated. WHT = Westhighland terrier

Figure 2 is an Immunoblot of SDS-PAGE of the two antibodies B7.3 and B18-4.3. Antibody B7.3 shows the expected size for heavy and light chain, while antibody B18-4.3 has two shorter heavy chain fragments, which is caused by a mutation in the gene of the heavy chain of B18-4.3.

Figure 3 shows a flow cytometry graph of biotinylated B18-4.3 loaded onto beads with avidin surface. Red line (second peak) shows the shift caused by goat-anti-mouse-Phycoerythrin bound to B18-4.3 indicating that the antibodies bound to the beads.

Figure 4 shows the binding of fluorescent beads to dog hair via antibody B18-4.3. a: 0.8$\mu$m streptavidin surface modified beads (coupled to FITC) bound via biotinylated B18-4.3 to Westhighland terrier hair. b: 1 $\mu$m Neutravidin coupled fluospheres bound via biotinylated B18-4.3 to Beagle hair, which was pretreated by washing with Tris-buffer (pH 8.8) containing 300 mM KBr at 60°C.

Figure 5 shows the analysis of hair by confocal microscopy. Antibody B18-4.3 was primarily bound to the edges of the hair flakes. Detection by goat-anti-mouse-Alexa 488.

Figure 6 illustrates the analysis of antigen structures recognized by antibodies B7.3 and B18-4.3. Westhighland terrier hair was washed with Tris-buffer (pH 8.8) containing 300 mM KBr and this wash fraction was concentrated by acetone precipitation. Enriched hair surface proteins (eHSP) were analysed by immunoblot. Antigens were detected by primary antibody B7.3 or B18-4.3 followed by goat-anti-mouse AP-labeled secondary antibody and staining with NBT/BCIP.

Figures 7a and b illustrate the binding of hair-specific antibodies B7.3 and B18-4.3 to dog, cat, horse and human hair.

Figure 8 demonstrates the intense binding of the antibody equipped microparticles to dog hair in comparison to the poor binding to human hair

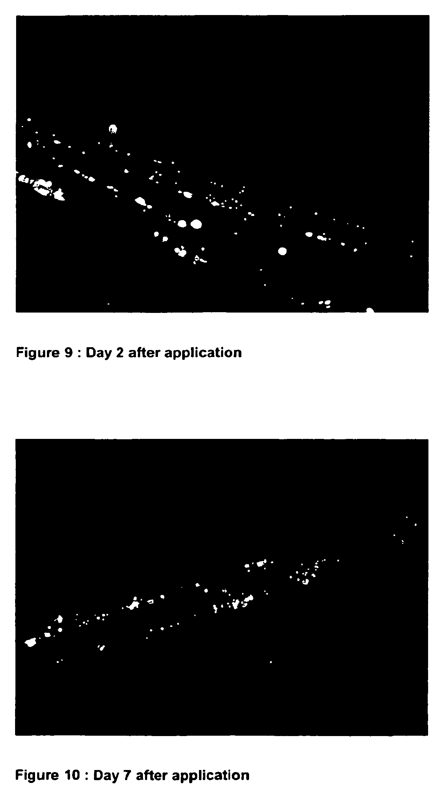

Figure 9 shows polystyrene microparticles with a mixture of the dog-hair specific antibodies B18-4.3 and B7.3 linked to their surfaces and containing a fluorescent dye, as they bind to beagle dog hair. The dye containing microparticles are used as binding indicators and become administered together with the same type of microparticles which contain an acaricide., This experiment was done to show the exemplary use of species-specific hair-binding antibodies and antibody equipped microparticles as controlled release drug carriers to protect dogs against ticks. The image shows the binding microparticles on some hair samples which were directly taken after the application, corresponding to 2 days after the infestation of the dog with ticks.

Figure 10 shows the microparticles as they bind to dog hair 7 days after the application of the formulation

Figure 11 a and b shows the microparticles as they bind to dog hair 14 days after the application of the formulation

Figure 12 a, b and c demonstrates that even after 42 days after the administration microparticles are still visible on the dog hair proving the persistence of the antibody mediated bonding of the microparticles to dog hair

Figure 13 demonstrates that even after 63 days after the administration of the formulations to dog hair microparticles are still visible on the hair surface

Generation and selection of monoclonal hair-specific antibodies

Animal hair-specific antibodies were generated through immunization of mice and subsequent use of hybridoma technology. Cut hair from Beagle was shredded using a homogeniser. Shredded hairs were approx. 200 $\mu$m in length.
Two mice were immunized with shredded Beagle hair. Maximal 80 $\mu$g hair sample (=20 $\mu$L shredded hair) was mixed with 40 $\mu$L GERBU adjuvant and 50 $\mu$L 1x PBS and used for immunization. Mice were immunized four times at October 11th, October 25th, November 3rd and November 28th and sacrificed at December 2nd. Spleen cells were isolated and fused to myeloma cells to generate hybridomas. Limited dilution was done by pipetting cells in increasing dilutions into 96-well microtiter plates. To screen for hybridoma clones producing hair-specific antibodies a new ELISA setup was established. Partially lysed hair (10 min in Shindai reagent at RT followed by homogenisation and resuspension in carbonate buffer) was coated onto low binding 96-well microtiter plates. After addition of the hybridoma cell culture supernatant, binding of specific antibodies was detected using a secondary goat-anti mouse (GAM) antibody conjugated to horseradish peroxidase (HRPO). In total 23 polyclonal hybridoma clones (B1-23) binding to hair samples were identified. However, since partially lysed hair was coated, both antibodies binding to antigens present inside the hair and to antigens on the hair surface were selected.
To identify binders recognizing structures on the hair surface, antibodies from monoclonal hybridoma lines were tested using the Eppendorf-based ELISA. 20-50 human or animal hairs were fixed with one drop of glue to the bottom of an Eppendorf tube and unspecific binding sites were blocked with 2% (w/v) BSA in PBS. Hybridoma supernatant or 10$\mu$g of purified monoclonal antibody was transferred to the tube and incubated for 30 minutes at room temperature. After washing specific binding was detected using a goat-anti mouse secondary antibody (labeled with HRPO) and incubation for 20 minutes at room temperature. Finally, binding was visualized using the 2,2'-azino-bis 3-ethylbenziazoline-6-sulfonic acid (ABTS) reagent as a substrate for HRPO. Colour development was measured after 30 seconds at OD 405.
Two monoclonal antibodies were identified by the Eppendorf-based ELISA. These antibodies, named B7.3 and B18-4.3, showing specific binding to the surface of animal hair were purified and characterized regarding their specificity to different hair samples.

Purification of monoclonal antibodies

Monoclonal antibodies B7.3 and B18-4.3 were purified from hybridoma culture supernatant via protein G or alternatively by precipitation of ultrafiltration. Yields of purified protein ranged from 0.1 to 1.1 mg/mL. Purified antibodies were used for subsequent assays.

Characterization of monoclonal antibodies

Antibody binding - Eppendorf-based ELISA

To verify qualitatively the binding of purified monoclonal antibodies B7.3 and B18-4.3 to the hair surface the Eppendorf-based ELISA was used. The antibodies B7.3 and B18-4.3 bind with specificity to Yorkshire terrier hair. Importantly, no binding to human hair was detected.

Several additional Eppendorf-based ELISA were performed using the following hair samples: human, horse, cat, Schnauzer/Labrador mix, Australian shepherd, Poodle before and after washing (3 samples), Yorkshire terrier before and after washing (4 samples), Westhighland terrier before and after washing (2 samples)

A summary of the Eppendorf-based ELISAs is presented in Table 1. The data show that both monoclonal antibodies bind specifically to the surface of the hair from five canine species. No binding was observed when human, horse or cat hair was used demonstrating the strong specificity of the selected antibodies for dog hair. It seems that the monoclonal antibody B18-4.3 possesses a stronger binding activity to canine hair than B7.3. Some minor variations are present between the different hair samples. Hair treatment prior to the performance of the ELISA reduces the binding activity of all tested antibodies, although bound antibodies are still present.

**Table 1: Results of the Eppendorf-based ELISA tests. - = no binding, + = low binding (OD < 1), ++ = medium binding (OD > 1 < 3), +++ = high binding (OD > 3), \* = after washing.**

| Antibody | Human | Horse | Cat | Schnauzer/ Labrador | Australian Shepherd | Poodle | | | Yorkshire Terrier | | | | Westhigh-land Terrier | |
|---|---|---|---|---|---|---|---|---|---|---|---|---|---|---|
| | | | | | | 1 | 2 | 3 | 1 | 2 | 3 | 4 | 1 | 2 |
| B7.3 | - | - | - | + | + | ++ \* | ++ + \* | ++ ++ | + \* | ++ \* | + \* | + \* | ++ + \* | ++ + ++ |
| B18-4.3 | - | - | - | +++ | +++ | ++ + \* | ++ + ++ | ++ + ++ | ++ + \* | ++ + ++ | ++ \* | ++ \* | ++ + ++ | ++ + ++ |

Antibody binding and functionality

Another type of ELISA was established to be able to compare the binding of the two antibodies B7.3 and B18-4.3 to hair quantitatively. Dog hair was cut into 2-5mm long pieces and dissolved in PBS (5 mg/ml). 200μl of this suspension was pipetted into each well of a 96-well microtiter filtration plate (Multiscreen HTS, membrane pore size 1.2 μm, Millipore). Buffer and substrate exchanges are done by centrifugation so that the hair in the microtiter filtration plate is not diminished in these procedures.

In preliminary experiments the maximal amount of antibody that is bound to 1 mg of hair (= well) was determined to be 5-10 ng (depending on dog race and antibody). Therefore experiments were mostly performed with 2.5 or 5 ng/well.

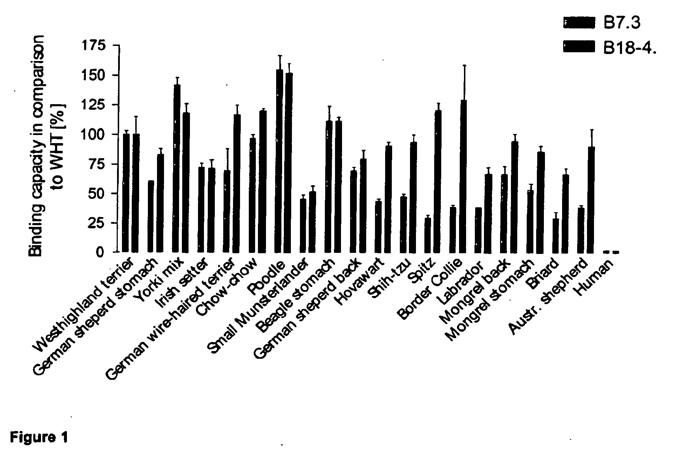

To check the binding of the two antibodies to hair of various dog races, a microtiter filtration plate ELISA was performed with hair of Westhighland terrier, German shepherd (hair from the stomach), Yorkshire terrier mix, Irish setter, German wired-hair terrier, Chow-chow, Poodle, small Munsterlander, Beagle, German shepherd (hair from the back region), Hovawart, Shih-tzu, Spitz, Border collie, Labrador, Mongrel (hair from the back), Mongrel (hair from the stomach), Briard, Australian shepherd. Also human hair was included in this test. All attained values were normalized in regard to Westhighland terrier (Figure 1).

Binding of the purified antibodies to the hair surface was also demonstrated by immunofluorescence imaging using a confocal microscope. For example, antibody B7.3 showed strong binding to Yorkshire terrier hair. No fluorescence was observed when applying only the secondary ALEXA564 dye labeled goat-anti mouse antibody, demonstrating the specificity of B7.3 to the hair surface. Treatment of hair with acetone prior to antibody application resulted in reduced fluorescence signals. Similar results were obtained when hair samples were washed with mild detergents.

Antibody integrity, stability and functionality

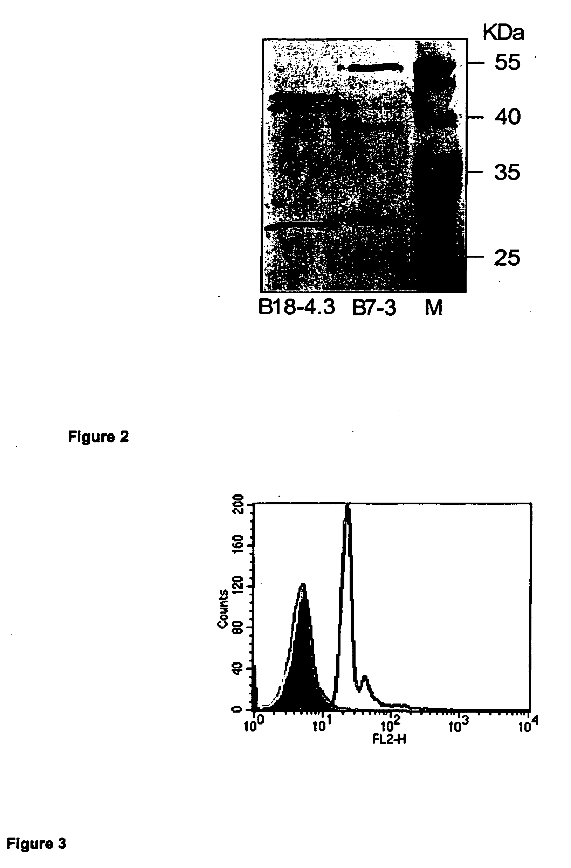

To verify the integrity of the antibody heavy and light chain, purified proteins were analysed by SDS polyacrylamide gel electrophoresis. While antibody B7.3 possesses an intact heavy and light chain of the expected size, antibody B18-4.3 has two heavy chain bands that are both smaller than the full-length heavy chain (approx 44 and 47 kDa instead of 55 kDa) (Figure 2). One of these mutated heavy chains is a result of a frameshift mutation at the end of the C2 domain which results in a premature stop codon. The origin of the other mutated heavy chain is not yet known. No degradation products of the B7.3 and B18-4.3 heavy chains were observed demonstrating the integrity of the purified antibodies.

To analyse the stability, binding of the monoclonal antibodies was carried out in the presence of isopropanol using the Eppendorf-based ELISA. The monoclonal antibody B7.3 showed stable binding in the presence of 30% (v/v) isopropanol over a period of four months at 4°C. Binding to the canine hair was still present when the antibody was kept at 4°C in 40% or 50% (v/v) isopropanol demonstrating the high stability of this protein. Moreover, the antibody B7.3 was stable at 22°C in the presence of 20% (v/v) isopropanol.

Stability of B18-4.3 was even better. The monoclonal antibody showed stable binding to the hair after storage in the presence of 40% (v/v) isopropanol over a period of four months at 4°C.

In addition, stability and functionality of monoclonal antibodies was tested in formulation solution containing 6% (v/v) Luviskol VA 64 W (BASF), 5% (v/v) Luviquat PQ 11 PN (BASF), 0.3% (v/v) Pluracare E400 PEG-8 (BASF), 0.2% (v/v) Q2-5220 Resin (DOW Coming) and 15% (v/v) isopropanol. After storage for 3 days at 4°C in the formulation antibody binding was tested using the Eppendorf-based ELISA indicating that B7.3 and B18-4.3 possess a high stability in the formulation.

Antibody binding capacity

To determine the maximum binding capacity, dilutions of B18-4.3 were tested in the Eppendorf-based ELISA using a defined number of Yorkshire terrier hairs (20 hairs, each 20mm long). The surface of the hairs in each Eppendorf tube corresponded to 3.15 mm$^2$. According to the ELISA 2.5-5.0 ng of antibody B18-4.3 binds to 3.15 mm$^2$ of hair surface.

Biotinylation of antibodies

To analyse the influence of binding partners on antibody functionality, purified monoclonal antibodies were biotinylated and subsequently bound to avidin beads, which was verified by FACS analysis (Figure 3). Binding to canine hair was analysed using the Eppendorf-based ELISA. Binding of the biotinylated antibodies B7.3 and B18-4.3 was detected via avidin conjugated to HRPO demonstrating that biotinylation and binding partners do not affect antibody functionality.

Antibody fusion to beads

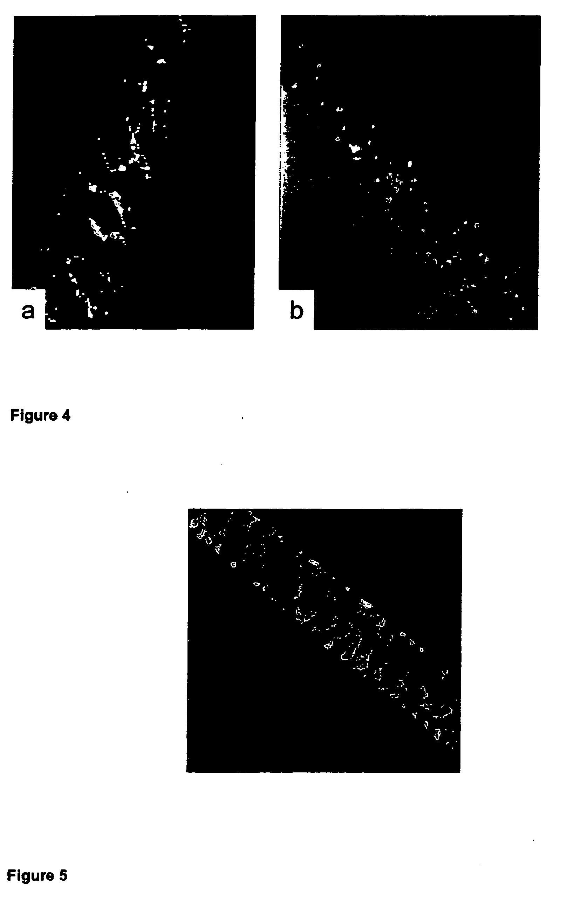

To analyse the binding of monoclonal antibody coupled to beads, hair from Westhighland terrier was pretreated with 1 x PBS containing 0.5 % (v/v) Tween 20 to remove contamination and not tightly fixed antigen structures. Subsequently, biotinylated antibody B18-4.3 (0.8 mg/mL) was applied and bound antibody was detected using streptavidin coated FITC fluorescent beads (Kisker). Bead size was 0.8 $\mu$m. In addition, biotinylated antibody B18-4.3 was coupled to streptavidin coated FITC fluorescent beads and the complex was applied to the hair sample to analyse binding (Figure 4a).

Experiments show that biotinylated B18-4.3 loaded on 1 $\mu$m Neutravidin coupled fluospheres (Invitrogen) shows high binding on Beagle hair even if the hairs were pretreated by washing with Tris-buffer (pH 8.8) containing 300 mM KBr at 60°C. This indicates the high stability of the antigen on the hair surface (Figure 4b).

In case of the 2 $\mu$m beads this effect was less pronounced which might indicate that added beads are bound by several antibodies present on the hair surface. Importantly, experiments with 10 $\mu$m beads were negative. No staining was observed because beads were lost during washing procedures.

Analysis of hair samples by confocal microscopy indicated that antibodies were primarily bound to the edges of the hair flakes (Figure 5).

Identification of the antibody binding site

Results presented in Table 1 indicate that at least a portion of the surface antigen can be removed by washing hair samples with mild detergents. Removal of antigen structures led to reduced antibody binding. However, antibody binding is still detectable demonstrating that a significant portion of the antigen remains connected to the hair surface.

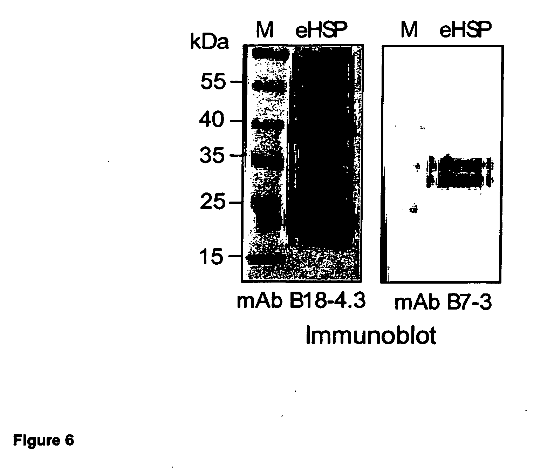

When hair of Westhighland terrier was washed with Tris-buffer (pH 8.8) containing 300 mM KBr and this wash fraction was concentrated, a preparation of enriched hair surface proteins (eHSP) was the result. These eHSP were analysed by SDS-PAGE and immunoblot, using either of the two antibodies B7.3 or B18-4.3. The result shows that B7.3 binds to structures that have a size of 32 KDa and 38 KDa while B18-4.3 detects three bands at 21 KDa, 28 KDa and 40 KDa (Figure 6). Therefore, the two antibodies have a different specificity.

Cloning of variable antibody chains

To secure the genetic information of the monoclonal antibodies, the variable antibody domains which determine the antibody binding specificity were cloned. The following steps were performed:

■ RNA isolation from the hybridoma clones

■ Reverse transcription into cDNA

■ Amplification of variable regions from antibody heavy and light chain by PCR

■ Fusion of both variable domains through a short linker fragment by splice overlap extension (SOE) PCR

■ Cloning of the single chain antibody (scFv) fragment into a bacterial vector

■ Transformation of bacteria

■ Plasmid isolation and sequencing

The integrity of scFvB7.3 and scFvB18-4 genes has been confirmed by sequencing.

ScFvB18-4 was tested for its binding to dog hair. Binding of scFvB18-4 alone or coupled to beads via biotin-avidin to hair samples was demonstrated by ELISA.

Binding of hair-specific antibodies B7.3 and B18-4.3 to dog, cat, horse and human hair

Each hair sample was measured three times by ELISA. The diagrams give mean and standard deviation of the samples. Two ELISAs were run in parallel and each diagram is the result of one ELISA. Where the same sample turns up in both diagrams (Westhighland terrier, European housecat), one hair sample was used to pipette both ELISA plates.

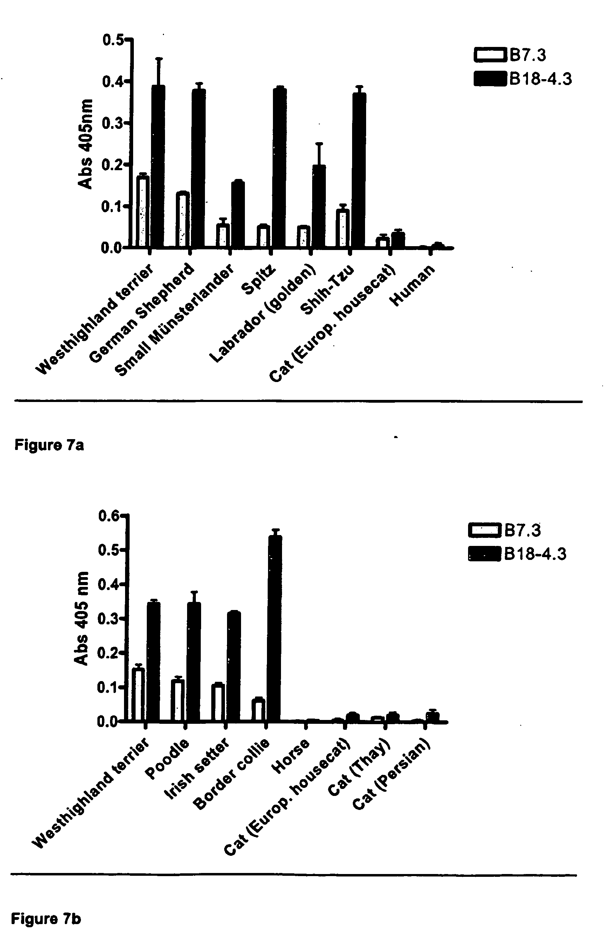

1 mg of hair was pippeted /well. Blocking was done with 2% (w/v) milk powder in PBS for 1h. As a first antibody either B7.3 or B18-4.3 was used at a concentration of 50ng/ml in PBS, where 100 $\mu$l antibody solution was used per well. Incubation was done at room temperature for 1h. Secondary antibody was a goat-anti-mouse (anti IgG, IgM and IgA) coupled to a peroxidase; the antibody was diluted 1:5000 in PBS and 100$\mu$l/well was used. Incubation was done at room temperature for 1h. Finally each well was incubated with 100$\mu$l of the substrate ABTS for 42 min. Reading of the resulting colour reaction was performed at 405nm. Between the various steps the plates were washed with twice 200 $\mu$l PBS-T. As a control three wells of each hair sample were incubated with an unspecific mouse antibody and otherwise treated as described above. The mean of each control was subtracted from the results with B7.3 and B18-4.3 of the same hair sample (Figure 7).

Generation of horse hair-specific antibodies

Horse hair-specific antibodies were generated through immunization of mice and subsequent use of hybridoma technology. Hair from horse was washed with PBS-T (1xPBS, Tween20 0,05%) shredded using a homogeniser. Shredded hairs were approx. 200 $\mu$m in length.

Two mice were immunized with washed, shredded horse hair. Maximal 80 $\mu$g hair sample (=20 $\mu$L shredded hair) was mixed with 40 $\mu$L GERBU adjuvant and 50 $\mu$L 1 x PBS and used for immunization. Mice were immunized six times at July 8th, July 22th, August 5th, August 18th, August 26th and September 8th and sacrificed at September 10th. Spleen cells were isolated and fused to myeloma cells to generate hybridomas. Polyclonal hybridoma clones were screened for production of horse-hair-specific antibodies with an ELISA setup as described above. Shredded horse hair in 1x PBS was coated onto low binding 96-well microtiter plates. After addition of the hybridoma cell culture supernatant, binding of specific antibodies was detected using a secondary goat-anti mouse (GAM) antibody conjugated to horseradish peroxidase (HRPO). For detection of antibody binding to hair ABTS-substrate was used and the absorption was measured at 405nm after 30 min incubation. In total 21 polyclonal hybridoma clones (HF 1-21) binding to hair samples were identified. However, since shredded hair was coated, both antibodies binding to antigens present inside the hair and to antigens on the hair surface were selected and single cells were cultivated further to generate monoclonals.

To identify binders recognizing structures on the hair surface, antibodies from monoclonal hybridoma lines were tested using the microtiter filtration plate ELISA (see Antibody binding and functionality pp10). 0,3mg hair of horse, dog, cat and human were added to separate wells on microtiter filtration plates and incubated in parallel with 100$\mu$l of hybridoma supernatant of each monoclonal to be tested. The test was performed as described above. Visualization of bound antibody was achieved by using a secondary antibody labeled with horseradish peroxidase (HRPO) and 2,2'-azino-bis 3-ethylbenziazoline-6-sulfonic acid (ABTS) reagent as a substrate for HRPO. Colour development was measured at 405 nm after 30 minutes incubation at room temperature. A clear binding specificity of antibody HF 17.6 to horse hair in comparison to dog, cat and human hair can be seen; the low signal is probably due to little antibody in the preparation as the test was performed only two days after selection of the monoclonals and few antibody producing cells existed.

**Table 2: Binding of monoclonal anti-horse hair antibody HF 17.6 to hair of four different species.** ELISA was performed as described above. Binding of antibody HF17.6 to hair of horse, dog, cat and human was evaluated. Values given are the absorbance at 405nm after 30 min incubation with ABTS with the background subtracted.

| Clone | Abs 405nm in ELISA on shredded HORSE hair | Abs 405nm in ELISA on shredded DOG hair | Abs 405nm in ELISA on shredded CAT hair | Abs 405nm in ELISA on shredded HUMAN hair |
|---|---|---|---|---|
| HF17.6 | 0.081 | 0.007 | 0 | 0 |

Generation of cat hair-specific antibodies

Cat hair-specific antibodies were generated through immunization of mice and subsequent use of hybridoma technology. Cut hair from European shorthair cat was shredded using a homogeniser. Shredded hairs were approx. 200 $\mu$m in length.

Two mice were immunized with shredded cat hair. Maximal 80 $\mu$g hair sample (=20 $\mu$L shredded hair) was mixed with 40 $\mu$L GERBU adjuvant and 50 $\mu$L 1x PBS and used for immunization. Mice were immunized six times at July 1st, July 15th, July 29th, August 12th, August 26th and September 1st and sacrificed at September 3rd. Spleen cells were isolated and fused to myeloma cells to generate hybridomas. Polyclonal hybridoma clones were screened for production of cat-hair-specific antibodies with an ELISA setup as described above. Shredded cat hair in 1x PBS was coated onto low binding 96-well microtiter plates. After addition of the hybridoma cell culture supernatant, binding of specific antibodies was detected using a secondary goat-anti mouse (GAM) antibody conjugated to horseradish peroxidase (HRPO). For detection of antibody binding to hair ABTS-substrate was used and the absorption was measured at 405nm after 30 min incubation. In total 73 polyclonal hybridoma clones (C2 1-73) binding to hair samples were identified. However, since shredded hair was coated, both antibodies binding to antigens present inside the hair and to antigens on the hair surface were selected. To select antibodies that bind selectively to cat hair, the same type of ELISA was performed 6 days later on human hair. The absorbance given in the following table demonstrates the superior binding to cat hair in comparison to human hair, indicating the production of cat-hair specific antibodies in the polyclonal hybridoma clones.

**Table 3: Binding of polyclonal anti-cat hair antibodies to cat or human hair was analysed by ELISA.** ELISA was performed as described above. Absorption of ABTS was measured after 30 min incubation and no background was subtracted. Abs = absorbance.

| Clone | Abs 405nm in ELISA on shredded cat hair | Abs 405nm in ELISA on shredded human hair |
|---|---|---|
| C2-2 | 0.89 | 0.26 |
| C2-32 | 0.93 | 0.24 |
| C2-43 | 0.99 | 0.27 |
| C2-45 | 0.92 | 0.22 |
| C2-47 | 1.15 | 0.2 |
| C2-50 | 0.86 | 0.22 |
| C2-68 | 0.93 | 0.2 |

**Example:**

In the following section, an example of an useful embodiment of the invention is provided. It is demonstrated how drug-containing microparticles, attached to dog fur via an antibody which selectively binds to dog hair, are able to protect an animal against pest insects - in this case ticks - for a prolonged period of time and without the side effect of skin irritation.

One common way to apply marketed topical paraciticide formulations to animal hair is to use so called "spot on" formulations in which a small volume of a drug containing solution is deployed onto the neck skin of the animal, having the disadvantage that it often causes severe irritation to the skin. The time period of protection against pest insects is restricted, for instance usually to four weeks in case of ticks. This applies also to other type of formulations with molecular dispersed acaricides.

It is shown that the unique property of the antibody - species selective binding to hair - is maintained when the antibody becomes coupled to a surface of a microparticle. It is demonstrated that the efficacy against ticks (akarizide efficacy > 90%) lasts for least three to four weeks. Reduced efficacy > 70% can be shown for additional four weeks and can be attributed to the sustained release properties of the particles. Also described is a useful method to link the antibodies to functional groups on the surfaces of the microparticles and a suitable application method to animal fur.

**A) Composition and Production of Drug-containing Microparticles**

The particles are made of polymers. They are non visible on fur, as their size is between 1-10$\mu$m. The active ingredient is encapsulated and released continuously by diffusion. The release can be controlled in a wide range by particle design (polymer type, molecular weight, additives). The particle surface is modified with functional groups, e. g. amine or carboxyl, which are necessary for the linking of the antibody.

In a preferred embodiment, the particles are compounded by the solvent evaporation technique. Therefore the active ingredient, in case of this example the pyrethroid Flumethrin (3-[2-Chloro-2-(4-chlorophenyl)ethenyl]-2,2-dimethylcyclopropanecarboxylic acid cyano(4-fluoro-3-phenoxyphenyl)methyl ester, CAS Nr. 69770-45-2), and the polymer, in this case polystyrene, are dissolved in dichloromethane. The emulsifier, a copolymer containing polymer segments which are compatible (miscible) with polystyrene and also carboxylic groups COOH which serve as the hydrophilic emulsifying moiety, is dispersed in water. Then the oil phase is dispersed in water with the help of an Ultra Turrax stirrer and the mixture is homogenized with a Microfluidizer. The droplet size is controlled by a microscope. The pH might be set to an alkaline range in order to facilitate the emulsification. The emulsion is then heated up to 60°C while stirring. At that temperature the dichloromethane evaporates. The emulsion is turned into a suspension, with the free COOH providing a hydrophilic surface. The particle size may be controlled by a mastersizer. The amount of active ingredient is analyzed by HPLC. In case of the given example, the Flumethrin content in the solid polystyrene microparticles is 18% m/m. The formulation code 7c_6+7_EE is given to this type of microparticle.

Microparticles loaded with a fluorescent dye, in this case Uvitex (2,5 thiophenediylbis(5-tert-butyl-1,3-benzoxazole), CAS No. 7128-64-5, 435nm) are made the same way. The active ingredient is replaced with the dye. The function of these particles is to visualize the antibody mediated bonding of the microparticles to the fur as they are easily detected with a fluorescence microscope.

In another embodiment, the microparticles are loaded by the soaking method. To 10my of a 15% dispersion of polystyrene microparticles, modified with surface COOH groups, 2,4ml of a dichloromethane phase, containing appropriate amounts of Flumethrin, are added. 24 hours of shaking at ambient room temperature follows. The solvent dichloromethane is evaporated afterwards at reduced pressure (150-50mbar). The dispersion becomes repeatedly centrifuged and the pellets washed with Ethanol/Water mixture to remove unencapsulated and surface bound Flumethrin. After renewed centrifugation, the pellets become redispersed in water and freeze dried. The microparticle encoded BU 163 contained 9% m/m of the drug.

## B) Linkage of the antibody to the Surface of the Microparticles

The coupling of the hair specific antibody to the drug delivering particle was achieved by the following method:

The carboxylate modified particles are activated by adding a specific amount of *N*-Ethyl-*N°*-(3-dimethylaminopropyl)carbodiimide (EDC; 2 mM). Then the activated form is stabilized by the addition of *N*-Hydroxysuccinimide (NHS; 5mM). The activated ester complex reacts with primary and secondary amines from the antibodies to give a covalent amide linkage between particle and antibody. In case of the microparticle type 7c_6+7_EE 80ml of a 2.18% *m/V* solution were mixed with 29ml of antibody solution, containing 69,6 mg of a antibody mixture (ratio B7.3 / B18-4.3 = 1 : 1). BU 163 became equipped with antibodies in a similar way.

The method is described in literature (Hermanson, G.T.: Bioconjugate techniques. Academic press, San Diego, 1996).

## C) Antibody-mediated Coupling of the Microparticles Specifically to Dog Fur

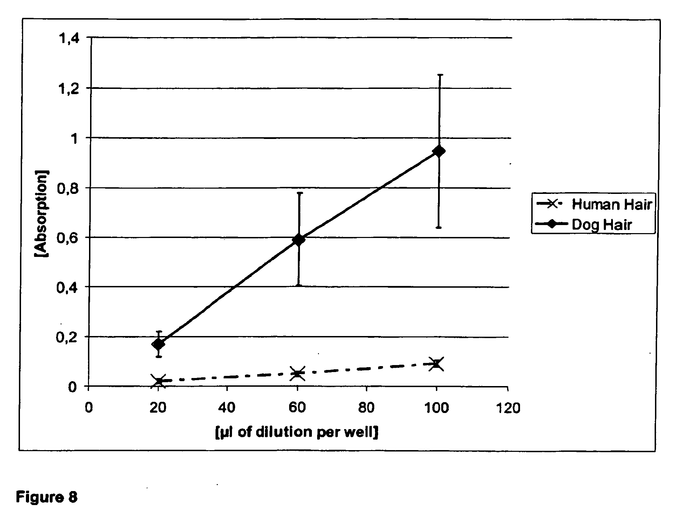

The proof that the microparticles equipped with the antibodies bind specifically to dog fur is given by spectroscopical detection of the active ingredient. A multi well plate is partly filled with equal amounts (mg) of dog and human hair. The bottom of the wells is a filter with a pore size of 30-40$\mu$m. Those hair containing wells are then filled with a suspension containing the antibody-carrying microparticles 7c_6+7_EE. The multi well plate is then centrifuged in order to remove the liquid and all of the non-bound particles by pressing the supernatant dispersion through the 30-40$\mu$m pores. The particles which are not binding to hair pass the filter as the particle size is only 1-10$\mu$m. Particles binding on hair remain attached to the hair inside the well. The hair is washed five times with a buffer containing 0,05% Tween 20. After each washing, the buffer is again removed by centrifugation. After this, acetonitrile - which dissolves the drug, but not the hair or the material of the multi-well plate - is added to the wells. The acetonitrile extracts the entire encapsulated drug out of the microparticle. The quantitative removal of the active ingredient was proven earlier by HPLC. A transparent multi well plate with no pores at the bottom of the wells is then placed under the hair containing multi well plate. It is made of a polymer which shows only very little UV absorbance. The acetonitrile is centrifuged into the wells of the transparent multi well plates. The active ingredient in the acetonitrile solution can be detected by UV light at 268nm. After calibration, the UV reader is able to detect the amount of drug present in the well, and therefore indirectly proof the attachment of the microparticles to dog or human hair. The more microparticles are linked to hair the more intense is the UV signal. This correlation is linearly dependent (Figure 8)

The plot (Figure 8) proves the binding of considerable amounts of the antibody equipped microparticles to dog hair. The dog hair was taken from seven different breeds (german-shepherd, collie-shepherd, terrier, spitz, schnauzer-labrador, mixed, collie, n=3).In contrast, only a small amount of the microparticles bind to human hair (three different women, untreated undyed hair, n=3), proving the conservation of the species selectivity of the antibodies' active hair binding domains when they become bound to the surfaces of microparticles. It is also demonstrated that the binding strength of the antibody to the animal hair is strong enough to sustain at least five washing cycles with buffer solution.

Instruments: ELISA: Synerg™ HT, BioTek; Microscope: Biozero BZ-8100E, Keyence

## C) Application to Animal Fur as a Spray Formulation

In one useful embodiment of a formulation, the antibody-equipped drug-containing microparticles are applied to the animal fur as a spray formulation.

In the given example, 6mg of encapsulated Flumethrin per kg bodyweight are applied to the dog. A beagle dog weights 11 kg on average. Therefore 367mg of the anti-body equipped microparticles 7c_6+7_EE containing 66mg of Flumethrin - as the drug concentration in the microparticles is 18% m/m - are applied to the animals. Depending on the active ingredient concentration of the particles and the amount of the drug needed, this value will vary. For the microparticles BU163 733mg are used, as the drug content is 9% m/m. 100mg of the fluorescent particles and the calculated amount of the drug-loaded microparticles are dispersed in a suitable amount of water. In case of this example, 30ml of water are used. A small amount of surfactant (in this example 0,01% Tween 20) is added to facilitate the spreading of the formulations on the sometimes fatty dog hair. The dispersions are filled into a common bottle, equipped with a pump spray head which is suitable to spray particle suspensions without blocking the valve, and are ready to use. The dispersions are sprayed evenly all over the dog fur, allowing a homogenous distribution of the microparticles onto the dog hair.

### E) In-vivo Study: Efficacy against ticks *(Rhipicephalus sanguineus)* on dogs

The objective of this study was to evaluate the effectiveness against ticks of two different spray formulations containing flumethrin loaded microcarriers bound to dog fur via antibodies on dogs experimentally infested with *Rhipicephalus sanguineus* (table 4).

Nine dogs were enrolled in this study in three groups of three dogs each. Each dog was housed in individual cages for the whole study period.

On study day (SD) -1 all dogs were sedated approximately 0.1 ml/kg BW Ketaminhydrochlorid (Ketamin®10%) in combination with approximately 0.1 ml/kg BW Xylazinhydrochlorid (Xylazin® 2%) i.m. During the sedation all dogs were laid in sternal or lateral recumbency on the floor and 50 *Rhipicephalus sanguineus* ticks (25♀, 25♂) were released onto the back of the dogs. The dogs were sleeping for 1h to 1.5 hours and therefore restrained from removing the ticks before they were able to attach.

On SD 0 tick infestation rate was determined without removal of the ticks by intensive adspection/palpation of the total body surface: Head, ears, neck, lateral areas, dorsal strip from shoulder blades to base of tail, tail and anal area, fore legs and shoulders, hind legs, abdominal area from chest to inside hind legs, feet. All live attached female ticks were counted and the dogs were randomly allocated to three study groups based on the tick counts.

After tick counting on day 0 treatment was performed. Dogs of the control group were left untreated and served as negative control group. The dogs of the two treatment groups were dosed once with the IVP (Investigational Veterinary Product). Each IVP had a volume of 30 ml and contained 66 mg flumethrin as active ingredient. Each dog received the total amount of 30 ml irrespective of the body weight. With the dog standing each dog was sprayed with the IVP evenly over the whole body.

**Table 4: Overview of the microparticle formulations** used in the in-vivo experiment, and information about dosing

| Group | IVP | Dosage [mg/kg BW] | Total volume applied |
|---|---|---|---|
| 1 | 7c_6+7_EE | 6mg/kgBW; average of dog weight 11 kg → 66mg/dog | 30 ml |
| 2 | BU 163 | | |
| 3 | Control | n.a. | n.a. |
| n.a. not applicable | | | |

On the day of the treatment (Study Day SD 0) the dogs were observed for adverse events at two and four hours post treatment. All dogs tolerated the treatment well. On SD 2 tick counts were performed in the same way as on SD 0 but with removal of the ticks. Ticks were identified as free or attached, engorged or unengorged, live or dead ticks. In regular intervals each dog was reinfested in the same manner and tick counting after 48 hours was performed as described. The chosen intervals were one, three, seven and nine weeks after treatment, e.g. tick infestation was done on SD 5, 20, 48 and 61, and tick counting two days later on SD 7, 22, 50 and 63.

Besides at each tick counting day (48 hours after infestation) 100 mg hair of each dog was taken in regular intervals. The fur samples were taken from all over the dog: shoulder, back, from the beginning of the tail, hip and investigated by fluorescence microscopy. Hair samples were taken at Days 2, 7, 14, 42 and 63 after the administration of the formulation.

Detailed general health observations were performed on all dogs during tick counting procedures. Special attention was paid to skin irritations. During the whole study period no skin irritations due to the treatment could be observed.

The tick counts were used to evaluate the efficacy of the IVP. Percent efficacy was calculated with a modified Abbott formula according to the recommendations for controlled tests described in the guideline EMEA/CVMP/005/00-Final:

$$\% \text{ Efficacy} = (N2-N1)\,/N2 \times 100$$

N1 = Geometric mean tick count for the group treated with IVP
N2 = Geometric mean tick count for the control group

"Tick count" is defined as "ticks representing a treatment failure". In case of determination of curative efficacy (SD 2) only live ticks were considered as treatment failure, whereas in case of determination of preventive efficacy (all remaining counting time points) live ticks and dead engorged ticks were considered as treatment failure.

An active ingredient is seen as highly effective if an efficacy of >90 % is achieved 48 h after treatment and weekly reinfestation. Efficacy should be given over a three to four week period.

**Table 5: Calculated efficacy [%]**

| group | SD 2 | SD 7 | SD 22 | SD 50 | SD 63 |
|---|---|---|---|---|---|
| Treatment group 1 | 97,2 | 100 | 97,4 | 78,3 | 56,2 |
| Treatment group 2 | 63,4 | 94 | 97,4 | 85,2 | 29,4 |

The minimum goal to show efficacy over 90% a period of three to four weeks was achieved (table 5). Given the high concentration of flumethrin in the particles, this form of application clearly protects the animals from skin irritation.

After 50 days post treatment reduced efficacy still is notable. The release of flumethrin from the microparticles after the study period of ≥50 days may not be sufficient to keep active levels of flumethrin on the skin of the animals. The better protection during the first weeks of the trial may be attributed to a certain part of free flumethrin and a higher diffusion rate from the particles in the early phase due to the high flumethrin concentration inside the matrix.

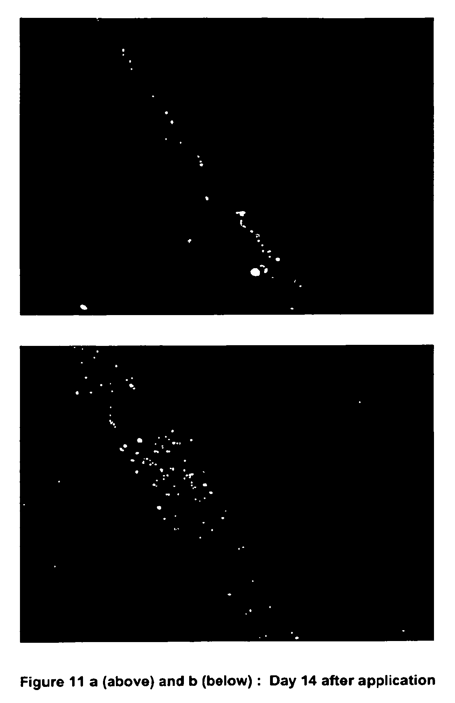

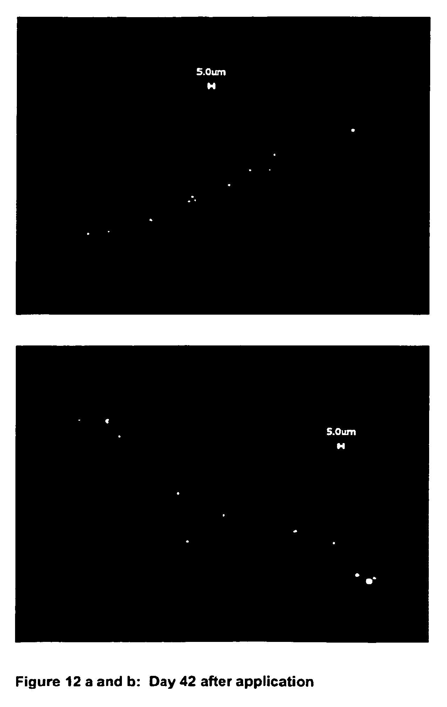

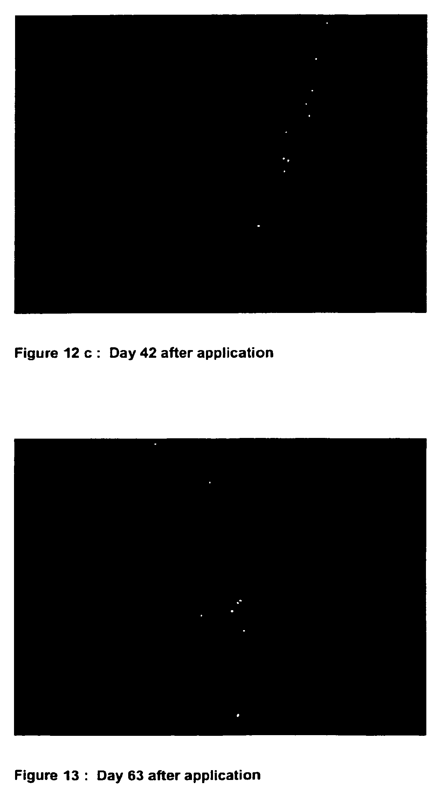

By fluorescence imaging, the persistent attachment of the microparticles to the dog fur can be detected It can be shown that microparticles remain attached to the hair via antibody binding for the whole period of the experiment (Figures 9 to 13). Note that the fluorescence-dye marked particles serve as indicator only and represent only 20% of the total number of microparticles in the formulations. With time, less particles can be detected, partially because they have fallen off, partially because the fluorescent dye bleaches out because of the constant exposure to daylight.

## F) Conclusions from the Example

The described example demonstrates one highly useful embodiment of the presented invention: The equipment of drug-containing microparticles which are suitable to control the release of the drugs over a wide range, with antibodies which specifically bind to animal hair (in this case dog hair). There are multiple advantages of this application of the invention:

- It allows to target paraciticides to a specific mammal species, thus considerable reducing the danger of cross-application or drug transfer, to e.g. humans or other mammal species, when the formulations are applied or when the different species interact, for instance by playing or licking.

- It allows to direct the drug to animal fur, therefore avoiding largely contact with the skin which reduces the potential for skin irritation or severe skin damage

- The universality of the principle of linking a single species-specific antibody to different microparticle surfaces via COOH, NH2 or other groups and deploy the microparticles to fur, allows to select a range of different microparticle types (different polymers containing different drugs with different release profiles) for a single formulation. It permits combinations of different type of microparticles in a dispersion and therefore to combine drugs in a single formulation which may not be chemically compatible otherwise.

- The use of suitable microparticles with appropriate drug release profiles allows to extent the efficacy of the drug to several weeks or months, depending on the particle and drug type, therefore providing the potential of considerably extending the efficacy period of common topically applied liquid pesticide and other drug formulations.

- The attachment of the microparticles to the animal hair is strong enough to overcome intense contact with water or even mild washing, e.g. when the animal is strolling around in rainy weather or is swimming in a pond for some time. In this case, regular formulations require often renewed application.

**EP 2 207 806 B1**

**Claims**

1. An antibody which selectively binds to the surface of hair of one animal species selected from the group of dog, cat, cattle, sheep, goat, camel, lama and horse.

2. The antibody according to claim 1 selected from the group comprising polyclonal antibodies, monoclonal antibodies or recombinant antibodies such as full-size antibody, dimeric secretory IgA, multimeric IgM and fragments thereof such as $F(ab')_2$-fragment, Fab-fragment, Fv-fragment, single chain Fv antibody (scFv), bispecific scfv, diabody, triabody, tetrabody, dingle domain antibody (dAb), minobody or molecular recognition unit (MRU).

3. The antibody according to claim 2 which is derived from hybridoma cells, synthetic, semi-synthetic, naive and immunocompetent phage libraries or ribosome display libraries, or by the generation of fully synthetic designer antibodies.

4. A drug delivery system comprising the antibody according to any one of claims 1 to 3.

5. A drug delivery system comprising the antibody of claims 1 to 3 which is in addition bound or attached to the surface of solid particles, thus selectively binding these particles to animal hair.

6. A drug delivery system according to claim 5 comprising of a formulation of the antibodies selectively binding to animal hair and particles containing an active ingredient, in such a way that the antibody is binding selectively to animal hair in a first step and the particles are bound to the antibody coupled to the animal hair In a second step.

7. A drug delivery system according to claim 6 comprising a formulation of the animal hair binding antibody linked or chemically bound to a suitable spacer/mediator/intermediate coupling agent and of particles, In such a way that the spacer/mediator/coupling agent carrying the antibody becomes selectively bound to animal hair via said antibody in a first step and that the particles become linked to the animal hair by binding to said spacer/mediator/coupling agent in a second step.

8. A drug delivery system according to any of the claims 5-7 in which the particles contain active ingredients or agents beneficial for the animal, namely drugs, insecticides, hair or skin caring agents, repellent agents which protect the treated animal from being attacked by harmful pest animals, e.g. insects, smell modifying agents or agents modifying the behavior of the treated animal or other non-treated individual animals which interact with the animal to which the treatment is applied.

9. A drug delivery system of any of the claims 5-8 where the particles contain active ingredients or agents beneficial for the animal in a concentration suitable for its therapeutic or beneficial purpose, namely 0.01 % to 99.9%.

10. A drug delivery system of any of the claims 5-9 where the particles have a diameter of 0.001 $\mu$m to 10$\mu$m, preferably 0.1 $\mu$m to 21$\mu$m.

11. A drug delivery system of any of the claims 5-10 where the microparticles contain a drug of the pyrethroid type which is administered to the fur of animals and delivers the pyrethroid over a prolonged period of time and without causing side effects, namely skin irritation.

**Patentansprüche**

1. Antikörper mit selektiver Bindung an die Oberfläche von Haar einer Tierart ausgewählt aus der aus Hund, Katze, Rind, Schaf, Ziege, Kamel, Lama und Pferd bestehenden Gruppe.

2. Antikörper nach Anspruch 1, ausgewählt aus der polyklonale Antikörper, monoklonale Antikörper und rekombinante Antikörper wie einen vollständigen Antikörper, dimeres sekretorisches IgA, multimeres IgM und Fragmente davon wie $F(ab')_2$-Fragment, Fab-Fragment, Fv-Fragment, Einzelketten-Fv-Antikörper (single chain Fv antibody, scFv), bispezifischen scFv, Diabody, Triabody, Tetrabody, Einzeldomänenantiкörper (single domain antibody, sdAb), Monobody oder Molecular Recognition Unit (MRU) umfassenden Gruppe.

3. Antikörper nach Anspruch 2, gewonnen aus Hybridomzellen, Bibliotheken synthetischer, halbsynthetischer, naiver

14

und immunkompetenter Phagen oder Ribosomdisplaybibliotheken, oder durch die Erzeugung vollsynthetischer Designer-Antikörper.

**4.** Arzneimittelverabreichungssystem, umfassend den Antikörper nach einem der Ansprüche 1 bis 3.

**5.** Arzneimittelverabreichungssystem, umfassend den Antikörper der Ansprüche 1 bis 3, welcher weiterhin an die Oberfläche fester Partikel gebunden ist oder daran anhaftet, wodurch diese Partikel selektiv an Tierhaar gebunden werden.

**6.** Arzneimittelverabreichungssystem nach Anspruch 5, umfassend eine Formulierung der selektiv an Tierhaar und einen Wirkstoff enthaltende Partikel gebundenen Antikörper, derart, dass der Antikörper in einem ersten Schritt selektiv an Tierhaar bindet und die Partikel in einem zweiten Schritt an den an das Tierhaar gekoppelten Antikörper gebunden werden.

**7.** Arzneimittelverabreichungssystem nach Anspruch 6, umfassend eine Formulierung des an ein geeignetes Spacer-/ Vermittler-/Zwischenglied-Kupplungsmittel chemisch gebundenen oder damit verbundenen Tierhaar bindenden Antikörpers und von Partikeln, derart, dass der Spacer/der Vermittler/das Kupplungsmittel, welcher/welches den Antikörper trägt, in einem ersten Schritt über diesen Antikörper selektiv an Tierhaar gebunden wird und dass die Partikel in einem zweiten Schritt durch Bindung an den Spacer/den Vermittler/das Kupplungsmittel an das Tierhaar gebunden werden.

**8.** Arzneimittelverabreichungssystem nach einem der Ansprüche 5-7, wobei die Partikel Wirkstoffe bzw. Mittel enthalten, die für das Tier von Nutzen sind, d.h. Arzneimittel, Insektizide, Haar- oder Hautpflegemittel, Repellenzien, die das behandelte Tier gegen einen Befall durch Schädlinge, z.B. Insekten, schützen, geruchsmodifizierende Mittel oder Mittel, die das Verhalten des behandelten Tieres oder anderer nicht behandelter einzelner Tiere, die mit dem Tier, bei dem die Behandlung durchgeführt wird, interagieren, modifizieren.

**9.** Arzneimittelverabreichungssystem nach einem der Ansprüche 5-8, wobei die Partikel Wirkstoffe oder für das Tier nützliche Mittel in einer für deren therapeutischen oder nützlichen Zweck geeigneten Konzentration enthalten, d.h. von 0,01% bis 99,9%.

**10.** Arzneimittelverabreichungssystem nach einem der Ansprüche 5-9, wobei die Partikel einen Durchmesser von 0,001 $\mu$m bis 10 $\mu$m, vorzugsweise 0,1 $\mu$m bis 2 $\mu$m, aufweisen.

**11.** Arzneimittelverabreichungssystem nach einem der Ansprüche 5-10, wobei die Mikropartikel ein Arzneimittel des Pyrethroidtyps enthalten, welches auf das Fell von Tieren aufgebracht wird und das Pyrethroid über einen längeren Zeitraum und ohne dass dabei Nebenwirkungen, d.h. eine Hautreizung, verursacht werden, verabreicht.

**Revendications**

**1.** Anticorps qui se fixe sélectivement à la surface de poils d'une espèce d'animal choisie dans le groupe constitué par le chien, le chat, le bovin, le mouton, la chèvre, le chameau, le lama et le cheval.

**2.** Anticorps selon la revendication 1, choisi dans le groupe constitué par les anticorps polyclonaux, les anticorps monoclonaux ou les anticorps recombinés tels que les anticorps pleine longueur, les IgA sécrétoires dimères, les IgM multimères et des fragments de celles-ci tels que le fragment F(ab')$_2$, le fragment Fab, le fragment Fv, les anticorps Fv à chaîne unique (scFv), les scFv bispécifiques, les di-anticorps, les tri-anticorps, les tétra-anticorps, les anticorps à domaine unique (dAb), les mini-anticorps ou les unités de reconnaissance minimale (MRU).

**3.** Anticorps selon la revendication 2, qui est dérivé de cellules d'hybridome, de banques de phages synthétiques, semi-synthétiques, naïfs et immunocompétents ou de banques de présentation de ribosomes, ou de la génération d'anticorps sur mesure totalement synthétiques.

**4.** Système d'administration de médicament comprenant l'anticorps selon l'une quelconque des revendications 1 à 3.

**5.** Système d'administration de médicament comprenant l' anticorps selon les revendications 1 à 3, qui est en outre fixé ou lié à la surface de particules solides, fixant ainsi sélectivement ces particules aux poils d'animaux.

**6.** Système d'administration de médicament selon la revendication 5, comprenant une formulation d'anticorps se fixant sélectivement aux poils d'animaux et des particules contenant un ingrédient actif, d'une manière telle que l'anticorps est fixé sélectivement aux poils d'animaux dans une première étape, et que les particules sont fixées à l'anticorps couplé aux poils d'animaux dans une deuxième étape.

**7.** Système d'administration de médicament selon la revendication 6, comprenant une formulation de l'anticorps se fixant aux poils d'animaux fixé ou lié chimiquement à un agent de couplage/médiateur/espaceur intermédiaire convenable et de particules, d'une manière telle que l'agent de couplage/médiateur/espaceur portant l'anticorps devienne fixé sélectivement aux poils d'animaux via ledit anticorps dans une première étape et que les particules deviennent fixées aux poils d'animaux après fixation audit agent de couplage/médiateur/espaceur dans une deuxième étape.

**8.** Système d'administration de médicament selon l'une quelconque des revendications 5-7, dans lequel les particules contiennent des ingrédients actifs ou des agents bénéfiques pour l'animal, à savoir des médicaments, des insecticides, des agents de soin de la peau ou du pelage, des agents répulsifs qui protègent l'animal traité contre une attaque par des animaux nuisibles néfastes, par exemple des insectes, des agents de modification des odeurs ou des agents modifiant le comportement de l'animal traité ou d'autres animaux individuels non traités qui interagissent avec l'animal auquel le traitement est appliqué.

**9.** Système d'administration de médicament selon l'une quelconque des revendications 5-8, dans lequel les particules contiennent des ingrédients actifs ou des agents bénéfiques pour l'animal selon une concentration convenable pour ses fins thérapeutiques ou bénéfiques, à savoir de 0,01% à 99,9%.

**10.** Système d'administration de médicament selon l'une quelconque des revendications 5-9, dans lequel les particules possèdent un diamètre allant de 0,001 $\mu$m à 10 $\mu$m, préférablement de 0,1 $\mu$m à 2 $\mu$m.

**11.** Système d'administration de médicament selon l'une quelconque des revendications 5-10, dans lequel les microparticules contiennent un médicament de type pyréthrinoïde, qui est administré au pelage des animaux et administre le pyréthrinoïde sur une période prolongée sans provoquer d'effets secondaires, à savoir une irritation de la peau.

**Figure 1**

**Figure 2**

**Figure 3**

**Figure 4**

**Figure 5**

mAb B18-4.3 · · · mAb B7-3

Immunoblot

**Figure 6**

**Figure 7a**

**Figure 7b**

**Figure 8**

Figure 9 : Day 2 after application

Figure 10 : Day 7 after application

Figure 11 a (above) and b (below) : Day 14 after application

5.0um

5.0um

Figure 12 a and b:  Day 42 after application

**Figure 12 c :  Day 42 after application**

**Figure 13 :  Day 63 after application**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3987161 A, Widder
- US 5597386 A, Igarashi
- US 6123934 A, Koyama

**Non-patent literature cited in the description**

- **HERMANSON, G.T.** Bioconjugate techniques. Academic press, 1996